# EUROPÄISCHE PATENTSCHRIFT

(11) **EP 0 577 557 B1**
(45) Veröffentlichungstag und Bekanntmachung des Hinweises auf die Patenterteilung: **22.11.1995**
(21) Anmeldenummer: 93810445.2
(22) Anmeldetag: 22.06.1993
(51) Int. Cl.: C07C 309/32, C11D 3/42

(54) **Hydrate des 4,4'-Bis-(2-sulfostyryl)-biphenyl-dinatrium oder -dikalium Salzes**
Hydrates of 4,4'-Bis-(2-sulfostyryl)-biphenyl-disodium or -dipotassium salts
Sels disodiques et dipotassiques d'hydrates de 4,4'-bis(2-sulfostyryl)-biphenyl

(30) Priorität: 30.06.1992 CH 2042/92
(43) Veröffentlichungstag der Anmeldung: 05.01.1994
(73) Patentinhaber: CIBA-GEIGY AG, 4002 Basel (CH)
(72) Erfinder: Ehlis, Thomas, Dr., D-7800 Freiburg (DE); Geoffroy, André, D-68440 Habsheim (FR); Marti, Erwin, Dr., CH-4054 Basel (CH); Zelger, Josef, CH-4125 Riehen (CH); Franke, Karlheinz, Dr., CH-4054 Basel (CH); Burkhard, Andreas, Dr., CH-4055 Basel (CH)

(56) Entgegenhaltungen:
- EP-A- 0 385 374
- CH-A- 594 617
- FR-A- 1 583 595

## Beschreibung

Die vorliegende Erfindung betrifft neue Hydrate des 4,4'-Bis-(2-sulfostyryl)-biphenyl-dinatrium- oder -dikalium Salzes, charakterisiert durch ihren Gehalt an Hydratwasser, die damit verbundenen Kristallformen, charakterisiert durch ihr Röntgenbeugungsdiagramm, Verfahren zu deren Herstellung, sowie deren Verwendung zur Herstellung konzentrierter Formulierungen optischer Aufheller.

Optische Aufheller werden in neuster Zeit bevorzugt in Form wässriger Lösungen oder Suspensionen in den Handel gebracht. Hierzu werden z.B. die feuchten Filterkuchen oder auch die trockenen Pulver mit Wasser aufgeschlämmt. Diese Suspensionen werden mit Dispergatoren und Verdickungsmitteln zur Erhöhung von Homogenität, Benetzbarkeit und Lagerstabilität versetzt. Zu diesen Hilfsstoffen setzt man häufig noch einen Elektrolyten hinzu. Trotz dieser Zusatzstoffe gibt es Grenzen für die Konzentration an optischem Aufheller, oberhalb deren die Suspensionen oft nicht mehr lagerstabil und schlecht dosierbar sind. Diese Grenzen sind oft nicht reproduzierbar, da sie von der jeweiligen Vorbehandlung abhängen.

Grund für die unterschiedlichen Eigenschaften des 4,4'-Bis-(2-sulfostyryl)-biphenyl-dinatrium Salzes ist das Auftreten von Anhydraten und Hydraten mit unterschiedlichen Kristallformen, wie sie z.B. bei der Herstellung gemäss DE-OS-17 93 482 und der anschliessenden Reinigung, mit Lauge und Oxidationsmittel bei hohen Temperaturen, anfallen. Als Beispiele für die Anzahl an gebundenen Wassermolekülen seien Verbindungen der Formel (I) genannt, 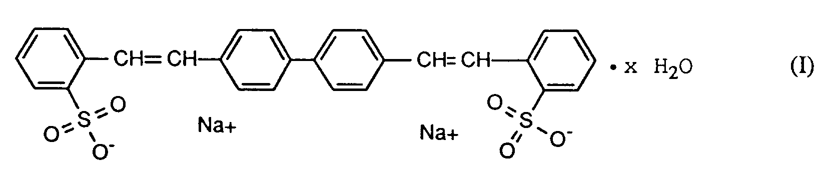
worin **x** eine Zahl von 0 bis 20, insbesondere 1, 2, 3, 5, 6, 7, 8, 9, 10, 11, 12, 13, 14 oder 15 bedeutet.

Der Ausdruck Aktivsubstanz bezieht sich im folgenden auf die Verbindung der Formel (I) worin **x** die Zahl 0 bedeutet.

Es wurde nun überraschenderweise gefunden, dass man lagerstabile Formulierungen an optischen Aufhellern mit Konzentrationen an Aktivsubstanz von über 30 Gew.% herstellen kann, bei denen sich die Viskosität in weiten Bereichen gezielt einstellen lässt, wenn ein bestimmtes Hydrate oder Mischungen von Hydraten des verwendeten optischen Aufhellers mit einer bestimmten Kristallform oder mehrerer bestimmten Kristallformen vorliegen.

Gegenstand der Erfindung sind somit neue Hydrate des 4,4'-Bis-(2-sulfostyryl)-biphenyl-dinatrium oder -dikalium Salzes, deren Kristallformen durch Röntgenbeugungsdiagramme im wesentlichen wie in Tabelle 1, 2, 3, 5 oder 6 charakterisiert sind.

Ein weiterer Gegenstand der Erfindung ist eine Mischung von Hydraten des 4,4'-Bis-(2-sulfostyryl)-biphenyl-dinatrium Salzes, die durch ein Röntgenbeugungsdiagramm im wesentlichen wie in Tabelle 4 charakterisiert ist.

Es treten hierbei Hydrate auf mit:
· einer plättchenförmigen Kristallform, die als p-Form bezeichnet wird und durch ein Röntgenbeugungsdiagramm im wesentlichen wie in Tabelle 1 charakterisiert ist,
· einer stäbchenförmigen Kristallform, die als i-Form bezeichnet wird und durch ein Röntgenbeugungsdiagramm im wesentlichen wie in Tabelle 2 charakterisiert ist,
· einer stäbchenförmigen Kristallform, die als j-Form bezeichnet wird und durch ein Röntgenbeugungsdiagramm im wesentlichen wie in Tabelle 3 charakterisiert ist,
· einer Mischung der Kristallformen i und j, die durch ein Röntgenbeugungsdiagramm, das im wesentlichen durch eine additive Überlagerung der Linien gemäss Tabellen 2 und 3, entsteht wie in Tabelle 4 gezeigt, charakterisiert ist,
· einer Kristallform, die als c-Form bezeichnet wird und durch ein Röntgenbeugungsdiagramm im wesentlichen wie in Tabelle 5 charakterisiert ist, sowie
· einer Kristallform von einem neuen Hydrat des 4,4'-Bis-(2-sulfostyryl)-biphenyl-dikalium Salzes,die durch ein Röntgenbeugungsdiagramm im wesentlichen wie in Tabelle 6 charakterisiert ist.

Hydraten der Formel (II) 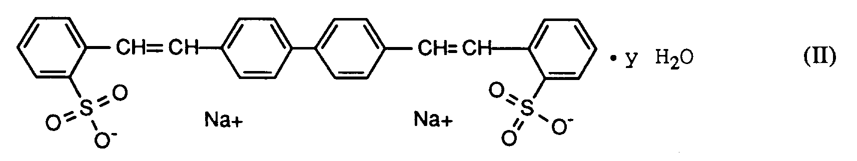
worin y eine Zahl zwischen 9 und 13 bedeutet.

Die stäbchenförmigen Kristallformen (i oder j) bestehen zum Grossteil aus einem oder mehreren Hydraten der Formel (III) 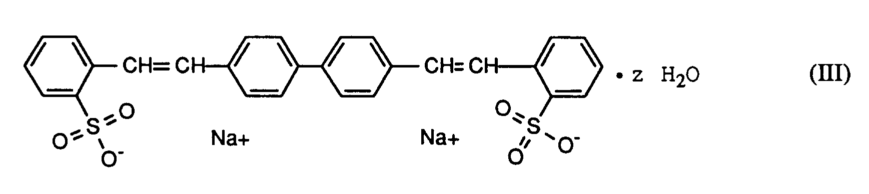
worin z eine Zahl zwischen 7 und 12 bedeutet.

Die Kristallform (c) besteht zum Grossteil aus einem Hydrate der Formel (IV)

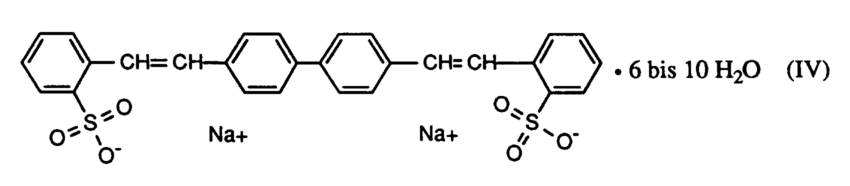

Die Kristallform von dem neuen Hydrat des 4,4'-Bis-(2-sulfostyryl)-biphenyl-dikalium Salzes besteht zum Grossteil aus dem Hydrat der Formel (V) 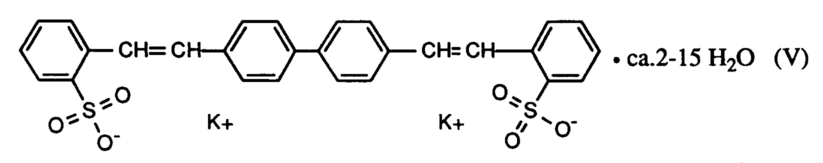
Die Menge an Hydratwasser wird üblicherweise bestimmt über
· Differenzthermoanalyse oder dynamischer Differenzkalorimetrie, wobei der Anteil des ungebundenen, also bei 0°C schmelzenden Wassers gemessen wird, oder dem
· Equilibrieren einer Probe unter einer bestimmten Luftfeuchte und Temperatur sowie der anschliessenden Bestimmung des Wassers durch Methoden wie Karl-Fischer Titration, Thermogravimetrie oder Trocknungsverlust bei erhöhter Temperatur.

Das plättchenförmige (p) Hydrat kann aus einer wässrigen Suspension der Hydrate in der Stäbchenform (i,j) oder einer Mischung aus den beiden salzfreien Hydraten bei einem Gehalt von höchstens 20 Gew.% an Aktivsubstanz durch mehrstündiges Rühren mit niedriger Scherkraft bei 5-45°C hergestellt werden. Zum Erreichen von Konzentrationen über 30 Gew.% an Aktivsubstanz können die so anfallenden Suspensionen über alle gebräuchlichen Methoden wie Zentrifugation, Vakuumverdampfung, Umkehrosmose oder Mikrofiltration aufkonzentriert werden.

Eine weitere Möglichkeit zur Herstellung besteht im Animpfen einer wässrigen Suspension eines beliebigen Hydrates des 4,4'-Bis-(2-sulfostyryl)-biphenyl-dinatrium Salzes mit plättchenförmigen (p) Impfkristallen. Diese Variante hat den Vorteil, dass jede Kristallform des Ausgangsmaterials und Konzentrationen von über 30 Gew.%, vorzugsweise von 30 bis 50 Gew.% Aktivsubstanz eingesetzt werden können und dadurch die erhaltene Suspension in der gewünschten Konzentration entsteht und nicht aufkonzentriert werden muss.

Günstigerweise wird dazu das aus der Synthese und der anschliessenden Reinigung anfallende 4,4'-Bis-(2-sulfostyryl)-biphenyl-dinatrium Salz in Wasser aufgeschlämmt, homogenisiert, die Impfkristalle zugesetzt und gerührt. Es kann hierbei kontinuierlich oder diskontinuierlich, so z.B. alle 2 Stunden für 15 Minuten, gerührt werden. Die Reaktionsdauer selbst ist von der Menge an eingesetzten Impfkristallen und der Rührgeschwindigkeit abhängig und kann zwischen 2 und 60 Stunden betragen.

Der Impfkristallanteil beträgt im allgemeinen zwischen 0,1 und 60 Gew.%, bevorzugt zwischen 1 und 50 Gew.% und besonders bevorzugt zwischen 1 und 30 Gew.%, bezogen auf den Gesamtgehalt an Aktivsubstanz. Wird jeweils nur ein Teil der erhaltenen Endverbindung durch neues Ausgangsprodukt ersetzt, kann die Umwandlung als halbkontinuierlicher oder kontinuierlicher Prozess durchgeführt werden.

Die Reaktionstemperatur zur Herstellung des plättchenförmigen (p) Hydrates beträgt hierbei im allgemeinen 5-45°C und bevorzugt 15-40°C.

In einer bevorzugten Massnahme wird die Stäbchenform in die Plättchenform umgewandelt, indem man eine wässrige Suspension der Stäbchenform mit dem Plättchen animpft. Die Impfkristalle sollten in Form von kleinen Kristallen, deren durchschnittliche Grösse 10 Mikron nicht überschreitet, benutzt werden. Dadurch kann der Impfkristallanteil merklich reduziert werden, z.B. bis zu 0.1 bis 5 Gew.%, bezogen auf den Gesamtgehalt an Aktivsubstanz. Bevorzugt wird bei der Umwandlung auf das Rühren verzichtet.

Eine andere Möglichkeit zur Herstellung der Plättchenform (p) besteht darin, das getrocknete Material mit Impfkristallen zu versetzen und bei 90-100% relativer Luftfeuchte für mehrere Tage bei 20-55°C in dünner Schicht zu belassen.

Die Darstellung des Hydrates mit der stäbchenförmigen Kristallform i, entsprechend der Tabelle 2, erfolgt durch Behandeln einer konzentrierten wässrigen Suspension eines beliebigen Hydrates des 4,4'-Bis-(2-sulfostyryl)-biphenyl-dinatrium Salzes für 2-12 Stunden, bei einer Temperatur von 70-130°C in einem geschlossenem Behälter oder in einer Rückflussapparatur und anschliessendes Abkühlen auf Raumtemperatur. Die Konzentration liegt dabei bevorzugt im Bereich von 30-60 Gew.% Aktivsubstanz.

In einer bevorzugten Massnahme wird die Darstellung des Hydrates mit der stäbchenförmigen Kristallform i, entsprechend der Tabelle 2, erfolgt durch Behandeln einer wässrigen Suspension, enthaltend 40 Gew.% eines beliebigen Hydrates des 4,4'-Bis-(2-sulfostyryl)-biphenyl-dinatrium Salzes für 4 Stunden, bei einer Temperatur von 95°C in einem geschlossenem Behälter und anschliessendes Abkühlen auf Raumtemperatur.

Die Darstellung des Hydrates mit der stäbchenförmigen Kristallform j, entsprechend der Tabelle 3, erfolgt durch Behandeln einer konzentrierten wässrigen Suspension eines beliebigen Hydrates des 4,4'Bis-(2-sulfostyryl)-biphenyl-dinatrium Salzes für 2 Stunden bis 4 Tage, bei einer Temperatur von 42-70°C, in einem geschlossenem Behälter oder in einer Rückflussapparatur und anschliessendes Abkühlen auf Raumtemperatur. Die Konzentration liegt dabei bevorzugt im Bereich von 30-60 Gew.% Aktivsubstanz.

In einer bevorzugten Massnahme wird die Darstellung des Hydrates mit der stäbchenförmigen Kristallform j, entsprechend der Tabelle 3, erfolgt durch Behandeln einer wässrigen Suspension, enthaltend 40 Gew.% 4,4'-Bis-(2-sulfostyryl)-biphenyl-dinatrium Salzes in der Plättchenform, für 4 Stunden, bei einer Temperatur von 95°C in einem geschlossenem Behälter und anschliessendes Abkühlen auf Raumtemperatur.

Im Gebiet von 70°C erhält man ein Gemisch der stäbchenförmigen Kristallformen i und j, bei 70-100°C erhält man hauptsächlich i- und bei 50-70°C hauptsächlich j-Form.

In allen Fällen kann die Reaktionszeit durch die Zugabe von Impfkristallen beschleunigt werden.

Das c-Hydrat kann aus einer wässrigen Suspension der Hydrate in der i- oder j-Stäbchenform oder in der p-Plättchenform hergestellt werden. Das Vorgehen ist wie folgt: Entfernen eines Teils des Wassers der Suspension durch ein geeignetes Verfahren, z.B. Zentrifugieren und anschliessendes Lagern für Zeiten von Stunden bis Tagen, entsprechend der eingestellten relative Feuchte von 52% bis gegen 100%. Suspensionen, hergestellt mit diesem c-Hydrat und stabilisiert durch Zusatz von Elektrolyt, sind lagerstabil bei 50°C.

Suspensionen der Hydrate i, j oder p können auch direkt in die c-Form umgewandelt werden und zwar durch Zusetzen von mindestens ca. 5 Gew.% NaCl bezogen auf das Gesamtgewicht des Slurries und Rühren bei Raumtemperatur oder bis 100°C und anschliessendes Abkuhlen.

Durch geeignete thermische Analyse wurde das c-Hydrat als 6 bis 10-Hydrat bestimmt. Ebenso ist eine eindeutige Charakterisierung durch die Bestimmung des Röntgenbeugungsmusters gegeben.

Die Kristallform des Hydrats des 4,4'Bis-(2-sulfostyryl)-biphenyl-dikalium Salzes kann mit wohlbekannten Methoden hergestellt werden.

Ein weiterer Gegenstand der Erfindung sind wässrige Formulierungen enthaltend 30-50 Gew.% 4,4'Bis-(2-sulfostyryl)-biphenyl-dinatrium Salz Aktivsubstanz in der plättchenförmigen Kristallform p. Diese Formulierungen bleiben fliessfähig, sind gut dosierbar und über Monate lagerstabil. Auch nach längerem Stehen bei Temperaturen zwischen 5-40°C sedimentieren sie nicht.

Um eine möglichst geringe Viskosität zu erreichen ist es günstig, dass die Korngrösse der Hauptmenge der Plättchen über 10 µm liegt und möglichst wenig Luft makroskopisch eingeschlossen wird.

Das suspendierte Produkt kann durch nochmaliges Homogenisieren von grösseren Agglomeraten befreit und im Unterdruck entlüftet werden.

Wässrige Formulierungen, enthaltend 30-50 Gew.% an Aktivsubstanz des 4,4'-Bis-(2-sulfostyryl)-biphenyl-dinatrium Salzes in den stäbchenförmigen Kristallformen i und j, sind ohne Zusatz von Formulierungshilfsmitteln hochviskos und eignen sich zur Herstellung von streichfähigen Pasten oder dazu, in solche eingearbeitet zu werden.

Durch Mischung der Hydrate mit den unterschiedlichen Kristallformen lässt sich so, ohne weitere Hilfsmittel, gezielt eine gewünschte Viskosität einstellen. Diesen Mischungen kann zur Stabilisierung der enthaltenen Hydrate ein Elektrolyt wie z.B. NaCl oder Na₂SO₄ beigemischt werden.

Ein besonderer Vorteil dieser Hydrate mit den unterschiedlichen Kristallformen liegt darin, gebrauchsfertige und lagerstabile Formulierungen unterschiedlichster Viskosität, ohne Zusatz von umweltbelastenden Formulierungshilfsmitteln zu ermöglichen.

Die so erhaltenen Formulierungen können jedoch auch die üblichen Formulierungshilfsmittel wie Dispergatoren, Gerüstbildner, Schutzkolloide, Stabilisatoren, Konservierungsmittel, Parfümierungsmittel sowie Sequestriermittel enthalten.

Als Dispergatoren kommen bevorzugt anionische wie die Kondensationsprodukte von aromatischen Sulfonsäuren mit Formaldehyd wie Ditolylethersulfonsäure, Naphthalinsulfonate oder Ligninsulfonate in Frage.

Als Gerüstbildner oder Schutzkolloide kommen z.B. modifizierte Polysaccharide, die sich von der Zellulose ableiten, oder Heteropolysaccharide wie Xanthan, Carboxymethylcellulose sowie Polyvinylalkohole (PVA), Polyvinylpyrrolidone (PVP), Polyethylenglycole (PEG) und Aluminium- oder Magnesiumsilikate in Frage. Sie werden üblicherweise in einem Konzentrationsbereich von 0,01 bis 2 Gew.% und bevorzugt 0,05 bis 0,5 Gew. %, bezogen auf das Gesamtgewicht der Formulierung, eingesetzt.

Als Hilfsmittel zur Stabilisierung können z.B. Ethylenglykol, Propylenglykol oder Dispergiermittel in einer Menge von 0,2 bis 5 Gew.% und bevorzugt 0,3 bis 2 Gew.%, bezogen auf das Gesamtgewicht der Formulierung, verwendet werden.

Als Konservierungsmittel finden z.B. Verbindungen wie 1,2-Benzisothiazolin-3-on, Formaldehyd oder Chloracetamid in einer Menge von 0,1 bis 1 Gew.% und bevorzugt 0,1 bis 0,5 Gew.%, bezogen auf das Gesamtgewicht der Formulierung, Verwendung.

Die so hergestellten konzentrierten Formulierungen können zum optischen Aufhellen von Papier oder Textilmaterial, wie z.B. in Waschmittel, eingesetzt werden. Sie werden dazu im allgemeinen auf die für die jeweilige Anwendung optimale Konzentration, durch den Zusatz von weiteren Hilfsmitteln oder Wasser, verdünnt.

Die folgenden Beispiele erläutern die Erfindung.

### Beispiele:

### Beispiel 1:

Es werden 100 ml einer Suspension, enthaltend 40 Gew.% 4,4'-Bis-(2-sulfostyryl)-biphenyl-dinatrium Salz Aktivsubstanz, in Form eines beliebigen Hydrates oder einer beliebigen Hydratmischung, für 4 Stunden auf 95°C in einem geschlossenem Bombenrohr erhitzt. Man erhält eine pastöse, streichfähige Formulierung.

Aus der mikroskopischen Analyse ergibt sich, dass nur noch die Stäbchenform vorliegt.

Das Röntgendiagramm, aufgenommen mit einer Guinierkamera in Transmissionsgeometrie und Cu-Kα1 Strahlung, entspricht dem Hydrat in der stäbchenförmigen Kristallform i, wie in Tabelle 2.

Zur Bestimmung des Gehaltes an Hydratwasser werden 200 mg des Hydratgemisches bei annähernd 100 % relativer Luftfeuchte und Raumtemperatur äquilibriert, auf 200°C erhitzt und der Gewichtsverlust gemessen. Es ergibt sich daraus ein Hydrat der Formel (III). 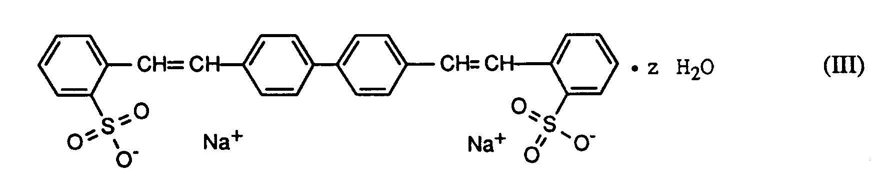
worin z eine Zahl zwischen 7 und 12 bedeutet.

### Beispiel 2:

Zur Herstellung der plättchenförmigen Impfkristalle werden 500 g der wie im Beispiel 1 erhaltenen wässrigen und salzfreien Aufschlämmung, enthaltend 40 Gew.% 4,4'-Bis-(2-sulfostyryl)-biphenyl-dinatrium Salz Aktivsubstanz in der Stäbchenform i, mit 500 ml deionisiertem Wasser vermischt und für ca. 12 Stunden bei 35°C mit 50 UpM gerührt. Die so erhaltene Suspension mit 20 Gew.% Anteil an Aktivsubstanz wird am Rotationsverdampfer bei 25 mbar und 35°C auf ca. 45 Gew.% eingeengt.

Aus der mikroskopischen Analyse ergibt sich, dass nur noch die Plättchenform p vorliegt.

Das Röntgendiagramm, aufgenommen mit einer Guinierkamera in Transmissionsgeometrie und Cu-Kα1 Strahlung, entspricht Tabelle 1.

Die Menge an gebundenem Hydratwasser wird durch den Trocknungsverlust bei 200°C gravimetrisch bestimmt, wobei die Probe zuerst bei Raumtemperatur und annähernd 100 % relativer Luftfeuchte äquilibriert wurde. Das vorhandene freie Wasser wird durch die dynamische Differenzthermoanalyse bestimmt. Dazu werden 18 mg der Hydratmischung auf -50°C gekühlt und die Menge an aufgenommener Wärme beim Aufheizen mit einer Aufheizrate von 10°C/Min gemessen.

Aus den Messungen ergibt sich ein Hydrat der Formel (II) 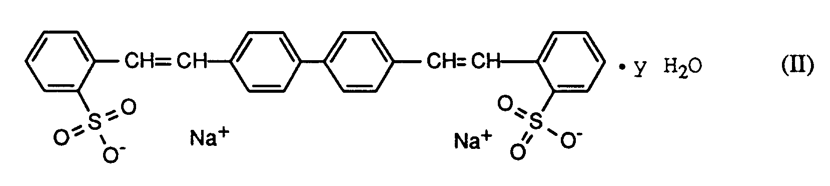
worin y eine Zahl zwischen 9 und 13 bedeutet.

### Beispiel 3:

Es werden 500 g der wie im Beispiel 1 erhaltenen wässrigen und salzfreien Aufschlämmung, enthaltend 40 Gew.% 4,4'-Bis-(2-sulfostyryl)-biphenyl-dinatrium Salz Aktivsubstanz in der Stäbchenform i, mit 500 ml deionisiertem Wasser vermischt und für ca. 12 Stunden bei 35°C mit 50 UpM gerührt. Die so erhaltene Suspension mit 20 Gew.% Anteil an Aktivsubstanz wird auf 0-5°C gekühlt und über eine Vakuumnutsche filtriert. Das resultierende Nutschgut hat einen Anteil von ca. 55 Gew.% Aktivsubstanz und wird mit deionisiertem Wasser auf ca. 45 Gew.% Aktivsubstanz verdünnt.

Aus der mikroskopischen Analyse ergibt sich, dass nur noch die Plättchenform p vorliegt.

Das Röntgendiagramm, aufgenommen mit einer Guinierkamera in Transmissionsgeometrie und Cu-Kα1 Strahlung, entspricht Tabelle 1.

### Beispiel 4:

Es werden 2 t einer 40 Gew.%^{igen} Suspension von 4,4'-Bis-(2-sulfostyryl)-biphenyl-dinatrium Salz, als plättchenförmige Impfkristalle, zu 8 t der aus der Produktion anfallenden 40 Gew.%^{igen} Suspension von 4,4'-Bis-(2-sulfostyryl)-biphenyl-dinatrium Salz, das auf 15-20°C thermostatisiert ist, gegeben. Diese Mischung wird für 60 Stunden bei dieser Temperatur langsam gerührt.

Aus der mikroskopischen Analyse ergibt sich, dass nur noch die Plättchenform p vorliegt, wobei der Hauptanteil der Plättchen grösser als 10 µm ist.

Das Röntgendiagramm, aufgenommen mit einer Guinierkamera in Transmissionsgeometrie und Cu-Kα1 Strahlung, entspricht Tabelle 1.

Die Analyse des Hydratgehaltes wird wie in Beispiel 2 beschrieben durchgeführt und ergibt eine Verbindung der Formel (II) 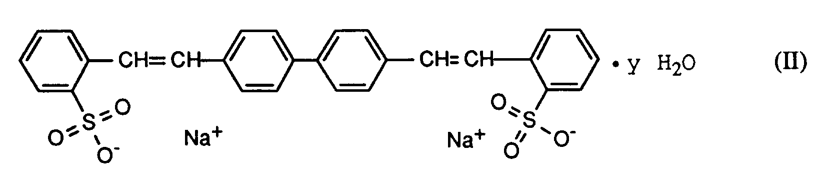
worin y eine Zahl zwischen 9 und 13 bedeutet.

### Beispiel 5:

Es werden 1,5 t einer 40 Gew.%^{igen} Suspension des aus der Produktion anfallenden 4,4'-Bis-(2-sulfostyryl)-biphenyl-dinatrium Salzes auf 15-20°C thermostatisiert, mit 1,5 t der in Beispiel 3 erhaltenen Suspension von plättchenförmigen Kristallen versetzt und für 12 Stunden bei dieser Temperatur langsam gerührt.

Aus der mikroskopischen Analyse ergibt sich, dass nur noch die Plättchenform p vorliegt, wobei der Hauptanteil der Plättchen grösser als 10 µm ist.

Das Röntgendiagramm, aufgenommen mit einer Guinierkamera in Transmissionsgeometrie und Cu-Kα1 Strahlung, entspricht Tabelle 1.

### Beispiel 6:

Es werden 100 ml der im Beispiel 4 erhaltenen Suspension, enthaltend 40 Gew.% 4,4'-Bis-(2-sulfostyryl)-biphenyl-dinatrium Salz Aktivsubstanz in der Plättchenform, für 4 Stunden in einem Gefäss mit Rückflusskühler auf 70°C erhitzt. Man erhält eine pastöse, streichfähige Formulierung.

Aus der mikroskopischen Analyse ergibt sich, dass nur noch die Stäbchenform vorliegt.

Das Röntgendiagramm, aufgenommen mit einer Guinierkamera in Transmissionsgeometrie und Cu-Kα1 Strahlung, entspricht dem Hydrat in der stäbchenförmigen Kristallform j, wie in Tabelle 3.

Zur Bestimmung des Gehaltes an Hydratwasser werden 200 mg des Hydratgemisches bei annähernd 100 % relativer Luftfeuchte und Raumtemperatur äquilibriert, auf 200°C erhitzt und der Gewichtsverlust gemessen. Es ergibt sich daraus ein Hydrat der Formel (III). 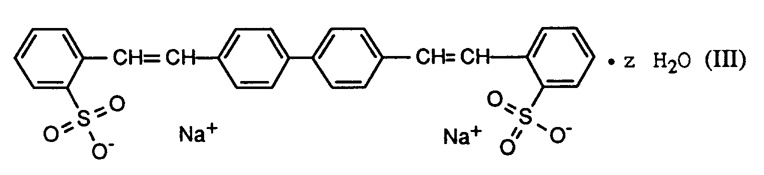
worin z eine Zahl zwischen 7 und 12 bedeutet.

### Beispiel 7:

Es werden je 100 g der in Beispiel 1 und 6 erhaltenen 40 Gew.%^{igen} Suspensionen der Stäbchenformen i und j vermischt. Man erhält eine pastöse, streichfähige Formulierung.

Das Röntgendiagramm, aufgenommen mit einer Guinierkamera in Transmissionsgeometrie und Cu-Kα1 Strahlung, entspricht der additiven Überlagerung der Linien gemäss Tabellen 2 und 3, wie in Tabelle 4 gezeigt.

### Beispiel 8:

Es werden 9,9 t der wie in Beispiel 3 und 4 dargestellten 40 Gew.%^{igen} Suspensionen des Hydrates in der plättchenförmigen Kristallform in einer Zahnkolloidmühle homogenisiert. Zu dieser Suspension werden 50 kg 1,2-Propylenglykol und 20 kg Rhodopol 23® sowie 30 kg Proxel GXL® gegeben und nochmals homogenisiert. Man erhält so 10 t einer wässrigen und fliessfähigen Formulierung von 4,4'-Bis-(2-sulfostyryl)-biphenyl-dinatrium Salz, die ca. 40 Gew.% Aktivsubstanz enthält und über Monate lagerstabil ist.

### Beispiel 9:

### A) Herstellung von Impfkristallen in in der Plättchen (p) Form:

In einem Reaktor werden 500g eines Slurries, enthaltend 40 Gew.% 4,4'-Bis-(2-sulfostyryl)-biphenyl-dinatrium Salz Aktivsubstanz, in Form eines beliebigen Hydrates oder einer beliebigen Hydratmischung, eingebracht und mit 200 U/min bei 15 bis 35°C gerührt. 500g deionisiertes Wasser werden zugegeben und der Trockengehalt kontrolliert (Sollwert=20 Gew.% Aktivsubstanz). Der 20%^{ige} Slurry wird für 15 bis 30 min auf 100°C (Rückfluss) erhitzt, wobei die Aktivsubstanz in Lösung geht. Innerhalb von 4 bis 6h wird auf 35°C abgekühlt und es wird dabei mit 200 U/min gerührt. Dabei kristallisiert die Aktivsubstanz in der i-Form (Stäbchen) wieder aus.

Mittels mikroskopischer Kontrolle wird der Umwandlungsprozess zur p-Form verfolgt. Die Temperatur wird dabei im Bereich 30 bis 36°C gehalten. Während des Prozesses (ca. 4 bis 10 h) nimmt die Viskosität des Slurries stark zu. Wenn keine Stäbchen (i,j-Form) mehr erkennbar sind, ist der Umwandlungsprozess abgeschlossen und die Aktivsubstanz liegt dann in der p-Form vor. Die Kristallgrösse ist Kleiner als 10 µm.

### B) Umwandlung der i-Kristallform in die p-Kristallform:

In einem Reaktor werden 900g eines Slurries, enthaltend 40 Gew.% 4,4'-Bis-(2-sulfostyryl)-biphenyl-dinatrium Salz Aktivsubstanz, in Form eines beliebigen Hydrates oder einer beliebigen Hydratmischung, eingebracht und mit 200 U/min durchgerührt und mindestens 2h auf 100°C erhitzt. Innerhalb von 1 bis 4h wird der Reaktorinhalt auf 35°C abgekühlt und bei dieser Temperatur thermostatiert. Die Aktivsubstanz liegt nun einheitlich in der i-Form vor.

180g eines 20%^{igen} Slurries, hergestellt wie unter Beispiel 9A) beschrieben, werden als Impfkristalle zugemischt und mit 200 U/min. weitergerührt. Aus dem Reaktor wird ein Muster entnommen und der Trockengehalt ermittelt (Sollwert=35,5 bis 36 Gew.%). Falls nötig, wird durch Wasserzugabe der Sollwert eingestellt.

Mittels mikroskopischer Kontrolle wird der Umwandlungsprozess verfolgt. Wenn keine Stäbchen (i-,j-Form) mehr erkennbar sind, ist der Umwandlungsprozess abgeschlossen.

Die Aktivsubstanz liegt dann in p-Form vor. Der Hauptanteil der Plättchen ist grösser als 10 µm. Der Slurry wird homogenisiert und, falls Luft eingeschlossen ist, durch Anlegen von Vakuum entlüftet.

### Beispiel 10:

Es werden 100g der wie im Beispiel 1 erhaltenen wässrigen und salzfreien Aufschlämmung, enthaltend 40 Gew % 4,4'-Bis-(2-sulfostyryl)-biphenyl-dinatrium Salz Aktivsubstanz in den Stäbchenform (i) für 20 Minuten bei 10°C mit 10,000 UpM zentrifugiert. Nach Entfernen des dekantierten Wassers bleibt ein pastösen Bodensatz zurück welcher bei 75% relativer Feuchte und Raumtemperatur breit verteilt auf einem Uhrglas für 10 Tage equilibriert wird.

Das Röntgendiagramm, aufgenommen mit einer Guinier-Kamera in Transmissions-geometrie und Cu-Kα1 Strahlung, entspricht dem Hydrat in der Kristallform (c), wie in Tabelle 5.

Zur Bestimmung des Hydratswassers werden 200mg des Hydrates bei annähernd 100% relative Feuchte und Raumtemperatur equilibriert und auf 200°C erhitzt. Es ergibt sich ein Hydrat der Formel

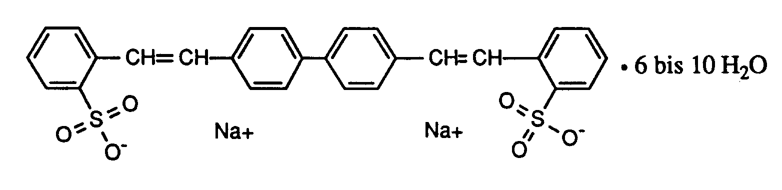

### Beispiel 11:

Es werden 100g der wie im Beispiel 7 erhaltenen Masse, enthaltend 40 Gew % 4,4'-Bis-(2-sulfostyryl)-biphenyl-dinatrium Salz Aktivsubstanz in den Stäbchenform (j), für 20 Minuten bei 10°C mit 10,000 UpM zentrifugiert. Nach Entfernen des dekantierten Wassers bleibt ein pastöse Bodensatz zurück welcher bei 75% relativer Feuchte und Raumtemperatur breit verteilt auf einem Uhrglas für 10 Tage equilibriert wird. Röntgendiagramm und Wassergehalt des erhaltene Produktes entsprechen denen des Produktes aus Beispiel 10.

### Beispiel 12:

Es werden 3 Liter der im Beispiel 4 erhaltenen Suspension, enthaltend 40 Gew % 4,4'-Bis-(2-sulfostyryl)-biphenyl-dinatrium Salz Aktivsubstanz in den Plättchenform (p) in einem Rückflusskolben mit 150g (5%) NaCl vermischt und für 2 Tage bei 50°C oder für 2 Stunde bei 100°C gerührt.

Das Röntgendiagramm, aufgenommen mit einer Guinier-Kamera in Transmissions-geometrie und Cu-Kα1 Strahlung, entspricht dem Hydrat in der Kristallform (c), wie in Tabelle 5.

**Tabelle 1**

| Hydrat des 4,4'-Bis-(2-sulfostyryl)-biphenyl-dinatrium Salzes in der plättchenförmigen Kristallform p. | | | |
|---|---|---|---|
| d-Wert [Å] | Intensität | d-Wert [Å] | Intensität |
| 17,9 | schwach | 3,77 | mittel |
| 13,8 | sehr schwach | 3,65 | sehr stark |
| 9,3 | mittel | 3,58 | schwach |
| 9,0 | sehr schwach | 3,51 | stark |
| 7,7 | schwach | 3,41 | sehr schwach |
| 7,5 | sehr schwach | 3,35 | schwach |
| 7,3 | sehr schwach | 3,21 | mittel |
| 6,9 | sehr schwach | 3,19 | stark |
| 6,3 | schwach | 3,14 | schwach |
| 6,1 | stark | 3,07 | schwach |
| 5,75 | sehr stark | 3,05 | schwach |
| 5,60 | schwach | 3,03 | schwach |
| 5,35 | stark | 3,02 | sehr schwach |
| 5,19 | sehr schwach | 2,98 | schwach |
| 5,04 | stark | 2,96 | sehr schwach |
| 4,81 | stark | 2,90 | mittel |
| 4,67 | schwach | 2,88 | schwach |
| 4,55 | schwach | 2,85 | sehr schwach |
| 4,50 | sehr schwach | 2,78 | sehr schwach |
| 4,35 | mittel | 2,68 | schwach |
| 4,12 | schwach | 2,65 | mittel |
| 4,00 | sehr schwach | 2,62 | schwach |
| 3,90 | stark | 2,56 | sehr schwach |
| 3,85 | stark | | |

**Tabelle 2**

| Hydrat des 4,4'-Bis-(2-sulfostyryl)-biphenyl-dinatrium Salzes in der stäbchenförmigen Kristallform i. | | | |
|---|---|---|---|
| d-Wert [Å] | Intensität | d-Wert [Å] | Intensität |
| 18,6 | sehr schwach | 4,49 | sehr schwach |
| 12,1 | schwach | 4,43 | schwach |
| 9,3 | sehr schwach | 4,37 | sehr schwach |
| 9,0 | sehr schwach | 4,25 | schwach |
| 8,8 | sehr schwach | 4,17 | schwach |
| 7,2 | schwach | 4,00 | sehr schwach |
| 6,8 | schwach | 3,95 | mittel |
| 6,7 | sehr stark | 3,93 | schwach |
| 6,4 | mittel | 3,86 | mittel |
| 5,97 | mittel | 3,73 | schwach |
| 5,78 | sehr schwach | 3,68 | schwach |
| 5,71 | schwach | 3,63 | schwach |
| 5,35 | schwach | 3,59 | schwach |
| 5,07 | mittel | 3,38 | sehr schwach |
| 4,90 | sehr schwach | 3,32 | schwach |
| 4,84 | sehr stark | 3,30 | schwach |
| 4,79 | stark | 3,19 | sehr schwach |
| 4,53 | sehr schwach | 3,00 | sehr schwach |

**Tabelle 3**

| Hydrat des 4,4'-Bis-(2-sulfostyryl)-biphenyl-dinatrium Salzes in der stäbchenförmigen Kristallform j. | | | |
|---|---|---|---|
| d-Wert [Å] | Intensität | d-Wert [Å] | Intensität |
| 19,8 | sehr schwach | 4,73 | sehr stark |
| 11,1 | mittel | 4,62 | schwach |
| 7,0 | schwach | 4,60 | stark |
| 6,9 | sehr stark | 4,40 | schwach |
| 6,4 | stark | 4,36 | sehr schwach |
| 6,3 | schwach | 4,25 | sehr schwach |
| 6,0 | sehr schwach | 4,20 | stark |
| 5,88 | schwach | 4,11 | stark |
| 5,71 | schwach | 3,88 | schwach |
| 5,63 | mittel | 3,86 | mittel |
| 5,55 | schwach | 3,75 | mittel |
| 5,29 | schwach | 3,69 | mittel |
| 5,17 | sehr schwach | 3,32 | sehr schwach |
| 5,13 | schwach | 3,25 | schwach |
| 5,01 | stark | 3,11 | schwach |
| 4,95 | mittel | 3,05 | schwach |
| 4,86 | sehr schwach | | |

**Tabelle 4**

| Mischung der Hydrate der stäbchenförmigen Kristallformen i und j des 4,4'-Bis-(2-sulfostyryl)-biphenyl-dinatrium Salzes. | | | |
|---|---|---|---|
| d-Wert [Å] | Intensität | d-Wert [Å] | Intensität |
| 19,7 | schwach | 4,60 | stark |
| 18,7 | schwach | 4,48 | sehr schwach |
| 11,1 | mittel | 4,40 | schwach |
| 7,0 | schwach | 4,37 | sehr schwach |
| 6,9 | stark | 4,26 | schwach |
| 6,6 | sehr stark | 4,21 | stark |
| 6,4 | sehr stark | 4,12 | stark |
| 6,3 | schwach | 3,87 | stark |
| 5,93 | (breit) mittel | 3,75 | mittel |
| 5,71 | mittel | 3,69 | mittel |
| 5,64 | mittel | 3,63 | sehr schwach |
| 5,56 | schwach | 3,59 | sehr schwach |
| 5,30 | mittel | 3,37 | sehr schwach |
| 5,13 | schwach | 3,32 | schwach |
| 5,06 | mittel | 3,30 | schwach |
| 5,01 | sehr stark | 3,25 | schwach |
| 4,96 | mittel | 3,18 | sehr schwach |
| 4,84 | (breit)stark | 3,12 | sehr schwach |
| 4,79 | stark | 3,06 | sehr schwach |
| 4,73 | stark | | |

**Tabelle 5**

| Hydrat des 4,4'-Bis-(2-sulfostyryl)-biphenyl-dinatrium Salzes in der Kristallform c | | | |
|---|---|---|---|
| d-Wert [Å] | Intensität | d-Wert [Å] | Intensität |
| 16,7 | schwach | 4,12 | sehr stark |
| 14,4 | stark | 4,01 | schwach |
| 12,0 | sehr schwach | 3,98 | sehr schwach |
| 10,8 | mittel | 3,85 | mittel |
| 8,3 | schwach | 3,72 | schwach |
| 7,2 | mittel | 3,64 | mittel |
| 6,6 | schwach | 3,56 | sehr schwach |
| 6,4 | schwach | 3,52 | sehr schwach |
| 6,1 | schwach | 3,49 | sehr schwach |
| 5,98 | sehr schwach | 3,36 | schwach |
| 5,51 | mittel | 3,31 | schwach |
| 5,42 | sehr stark | 3,29 | schwach |
| 5,25 | schwach | 3,21 | sehr schwach |
| 5,18 | schwach | 3,14 | schwach |
| 4,90 | schwach | 3,06 | sehr schwach |
| 4,85 | schwach | 2,98 | sehr schwach |
| 4,75 | mittel | 2,93 | schwach |
| 4,71 | schwach | 2,86 | schwach |
| 4,65 | schwach | 2,81 | sehr schwach |
| 4,58 | stark | 2,76 | sehr schwach |
| 4,32 | mittel | 2,71 | sehr schwach |

**Tabelle 6**

| Hydrat des 4,4'-Bis-(2-sulfostyryl)-biphenyl-dikalium Salzes | | | |
|---|---|---|---|
| d-Wert [Å] | Intensität | d-Wert [Å] | Intensität |
| 15,9 | sehr stark | 4,91 | schwach |
| 15,1 | stark | 4,80 | sehr schwach |
| 12,8 | sehr schwach | 4,72 | schwach |
| 11,5 | schwach | 4,67 | sehr schwach |
| 10,5 | stark | 4,48 | sehr schwach |
| 7,5 | sehr schwach | 4,41 | stark |
| 6,4 | mittel | 4,39 | mittel |
| 6,1 | stark | 4,25 | sehr schwach |
| 5,82 | sehr schwach | 4,22 | sehr schwach |
| 5,75 | sehr schwach | 4,11 | sehr schwach |
| 5,67 | schwach | 4,05 | sehr schwach |
| 5,61 | schwach | 3,97 | schwach |
| 5,54 | schwach | 3,81 | mittel |
| 5,47 | mittel | 3,77 | schwach |
| 5,43 | sehr schwach | 3,72 | schwach |
| 5,29 | sehr schwach | 3,69 | schwach |
| 5,01 | sehr stark | | |

## Patentansprüche

1. Hydrat des 4,4'-Bis-(2-sulfostyryl)-biphenyl-dinatrium oder -dikalium Salzes, dessen Kristallform durch ein Röntgenbeugungsdiagramm im wesentlichen wie in Tabelle 1, 2,3,5 oder 6 charakterisiert ist oder eine Mischung von Hydraten des 4,4'-Bis-(2-sulfostyryl)-biphenyl-dinatrium, deren Kristallformen durch ein Röntgenbeugungsdiagramm charakterisiert sind, das im wesentlichen durch die additive Überlagerung der Linien gemäss Tabellen 2 und 3 ertsteht, wie in Tabelle 4 gezeigt.

2. Hydrat in der plättchenförmigen Kristallform des 4,4'-Bis-(2-sulfostyryl)-biphenyl-dinatrium Salzes gemäss Anspruch 1 charakterisiert durch ein Röntgenbeugungsdiagramm im wesentlichen wie in Tabelle 1.

3. Hydrat in der stäbchenförmigen Kristallform des 4,4'-Bis-(2-sulfostyryl)-biphenyl-dinatrium Salzes gemäss Anspruch 1 charakterisiert durch ein Röntgenbeugungsdiagrarnm im wesentlichen wie in Tabelle 2 oder 3 oder eine Mischung aus den beiden stäbchenförmigen Kristallformen entsprechend einer additiven Überlagerung der Linien gemäss Tabellen 2 und 3, wie in Tabelle 4.

4. Hydrat in der Kristallform c des 4,4'-Bis-(2-sulfostyryl)-biphenyl-dinatrium Salzes gemäss Anspruch 1 charakterisiert durch ein Röntgenbeugungsdiagramm im wesentlichen wie in Tabelle 5.

5. Hydrat des 4,4'-Bis-(2-sulfostyryl)-biphenyl-dinatrium Salzes gemäss Anspruch 1 charakterisiert durch ein Röntgenbeugungsdiagramm im wesentlichen wie in Tabelle 6.

6. Hydrat der plättchenförmigen Kristallform (p) des 4,4'-Bis-(2-sulfostyryl)-biphenyl-dinatrium Salzes gemäss Anspruch 2, der Formel (II) 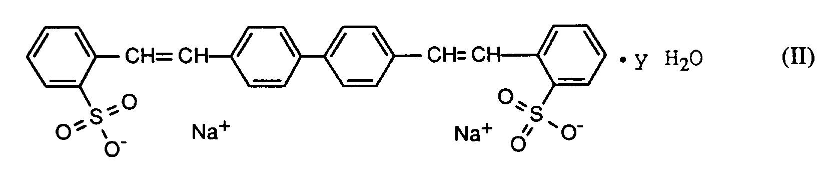 worin y eine Zahl zwischen 9 und 13 bedeutet.

7. Hydrat der stäbchenförmigen Kristallform des 4,4'-Bis-(2-sulfostyryl)-biphenyl-dinatrium Salz gemäss Anspruch 3, der Formel (III) 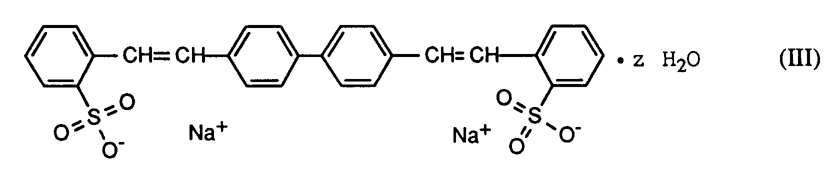 worin z eine Zahl zwischen 7 und 12 bedeutet.

8. Hydrat der Kristallform des 4,4'-Bis-(2-sulfostyryl)-biphenyl-dinatrium Salz gemäss Anspruch 4, der Formel (IV)

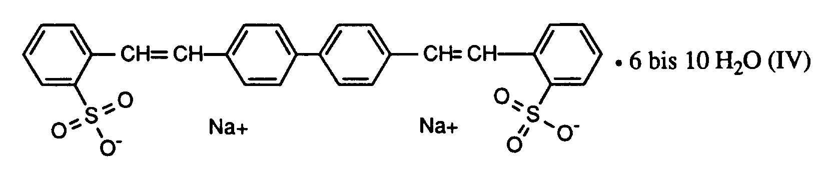

9. Hydrat des 4,4'-Bis-(2-sulfostyryl)-biphenyldikalium Salzes gemäss Anspruch 5, der Formel (V)

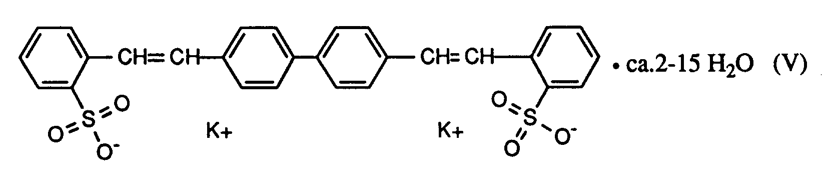

10. Verfahren zur Herstellung der plättchenförmigen (p) Kristallform gemäss Anspruch 2, dadurch gekennzeichnet, dass man eine wässrige Suspension von 4,4'-Bis-(2-sulfostyryl)-biphenyl-dinatrium Salz mit einem Gehalt an Aktivsubstanz von höchstens 20 Gew.%, in der Form von Hydraten mit einem Röntgenbeugungsdiagramm im wesentlichen wie in Tabelle 2,3 oder 4, für mehr als 4 Stunden bei 5 bis 45°C mit geringer Scherkraft rührt.

11. Verfahren zur Herstellung der plättchenförmigen (p) Kristallform gemäss Anspruch 2, dadurch gekennzeichnet, dass man eine wässrige Suspension eines beliebigen Hydrates des 4,4'-Bis-(2-sulfostyryl)-biphenyl-dinatrium Salzes mit plättchenförmigen Impfkristallen versetzt.

12. Verfahren zur Herstellung der plättchenförmigen (p) Kristallform gemäss Anspruch 11, dadurch gekennzeichnet, dass man eine wässrige Suspension mit einem Anteil von 30-50 Gew.% an Aktivsubstanz verwendet.

13. Verfahren zur Herstellung der plättchenförmigen (p) Kristallform gemäss Anspruch 11 oder 12 dadurch gekennzeichnet, dass man
a) das aus der Herstellung anfallende 4,4'-Bis-(2-sulfostyryl)-biphenyl-dinatrium Salz homogenisiert,
b) Impfkristalle zusetzt und
c) rührt.

14. Verfahren zur Herstellung der plättchenförmigen (p) Kristallform gemäss einem der Ansprüche 11 bis 13, dadurch gekennzeichnet, dass der Impfkristallanteil zwischen 0,1 und 60 Gew.%, bezogen auf den Gesamtgehalt an 4,4'-Bis-(2-sulfostyryl)-biphenyl-dinatrium Salz, beträgt.

15. Verfahren zur Herstellung der Verbindung der plättchenförmigen (p) Kristallform gemäss Anspruch 14, dadurch gekennzeichnet, dass der Impfkristallanteil zwischen 1 und 50 Gew.%, bezogen auf den Gesamtgehalt an 4,4'-Bis-(2-sulfostyryl)-biphenyl-dinatrium Salz, beträgt.

16. Verfahren zur Herstellung der Verbindung der plättchenförmigen (p) Kristallform gemäss Anspruch 15, dadurch gekennzeichnet, dass der Impfkristallanteil zwischen 1 und 30 Gew.%, bezogen auf den Gesamtgehalt an 4,4'-Bis-(2-sulfostyryl)-biphenyl-dinatrium Salz, beträgt.

17. Verfahren zur Herstellung der plättchenförmigen (p) Kristallform gemäss einem der Ansprüche 11 bis 16 , dadurch gekennzeichnet, dass bei einer Temperatur von 5-45°C gearbeitet wird.

18. Verfahren zur Herstellung der plättchenförmigen (p) Kristallform gemäss Anspruch 17, dadurch gekennzeichnet, dass bei einer Temperatur von 15-40°C gearbeitet wird.

19. Verfahren zur Herstellung der plättchenförmigen (p) Kristallform gemäss Anspruch 13, dadurch gekennzeichnet, dass man diskontinuierlich rührt

20. Verfahren zur Herstellung der plättchenförmigen (p) Kristallform gemäss Anspruch 11, dadurch gekennzeichnet, dass man
a) das aus der Herstellung anfallende 4,4'-Bis-(2-sulfostyryl)-biphenyl-dinatrium Salz zu einer Suspension mit 30-50 Gew.% Aktivsubstanz aufschlämmt und homogenisiert,
b) 1-50 Gew.% plättchenförmige Impfkristalle, bezogen auf den Gesamtgehalt an 4,4'-Bis-(2-sulfostyryl)-biphenyl-dinatrium Salz, zusetzt,
c) bei 15-40°C für 2-60 Stunden rührt,
d) nochmals homogenisiert und
e) durch Anlegen eines Unterdruckes entlüftet.

21. Verfahren zur Herstellung der plättchenförmigen (p) Kristallform gemäss Anspruch 11 oder 12, dadurch gekennzeichnet, dass man eine wässrige Suspension der Stäbchenform gemäss Anspruch 3 mit 0,1 bis 5 Gew.% des Plättchenforms gemäss Anspruch 2 animpft, bezogen auf den Gesamtgehalt der Aktivsubstanz.

22. Verfahren zur Herstellung der plättchenförmigen (p) Kristallform gemäss Anspruch 21 dadurch gekennzeichnet, dass die Impfkristalle eine durchschnittliche Grösse, die 10 µm nicht überschreitet, haben.

23. Verfahren zur Herstellung der plättchenförmigen (p) Kristallform gemäss Anspruch 21 oder 22 dadurch gekennzeichnet, dass man bei der Umwandlung auf das Rühren verzichtet.

24. Verfahren zur Herstellung der plättchenförmigen (p) Kristallform gemäss Anspruch 2, dadurch gekennzeichnet, dass man getrocknetes Ausgangsmaterial mit Impfkristallen vermischt und über mehrere Tage bei 90-100% relativer Luftfeuchte bei 20-55°C in dünner Schicht belässt.

25. Verfahren zur Herstellung der stäbchenförmigen Kristallform gemäss Anspruch 3 und Tabelle 2, dadurch gekennzeichnet, dass man eine wässrige Suspension eines beliebigen Hydrates des 4,4'-Bis-(2-sulfostyryl)-biphenyl-dinatrium Salzes für 2-12 Stunden auf eine Temperatur von über 70-130°C erhitzt und danach auf Raumtemperatur abkühlt.

26. Verfahren zur Herstellung der stäbchenförmigen Kristallform gemäss Anspruch 25, dadurch gekennzeichnet, dass man eine wässrige Suspension, enthaltend 40 Gew.% eines beliebigen Hydrates des 4,4'-Bis-(2-sulfostyryl)-biphenyl-dinatrium Salzes für 4 Stunden, bei einer Temperatur von 95°C in einem geschlossenem Behalter behandelt und danach auf Raumtemperatur abkühlt.

27. Verfahren zur Herstellung der stäbchenförmigen Kristallform gemäss Anspruch 3 und Tabelle 3, dadurch gekennzeichnet, dass man die wässrige Suspension eines beliebigen Hydrates des 4,4'-Bis-(2-sulfostyryl)-biphenyl-dinatrium Salzes für 2 Stunden bis 4 Tage auf eine Temperatur von 42-70°C erhitzt und danach auf Raumtemperatur abkühlt.

28. Verfahren zur Herstellung der stäbchenförmigen Kristallform gemäss Anspruch 3 und Tabelle 3, dadurch gekennzeichnet, dass man die wässrige Suspension enthaltend 40 Gew.% 4,4'-Bis-(2-sulfostyryl)-biphenyl-dinatrium Salzes in der Plättchenform, für 4 Stunden, bei einer Temperatur von 95°C in einem geschlossenem Behalter behandelt und danach auf Raumtemperatur abkühlt.

29. Verfahren zur Herstellung des Hydrats in der Kristallform (c) des 4,4'-Bis-(2-sulfostyryl)-biphenyl-dinatrium Salzes gemäss Anspruch 4 und Tabelle 5, dadurch gekennzeichnet, dass man einen Teil des Wassers einer wässrige Suspension der Hydrate in der (i-) oder (j-) Stäbchenform oder in der p-Plättchenform entfernt und anschliessend für Zeiten von Stunden bis Tagen, entsprechend der eingestellten relative Feuchte von 52% bis gegen 100% lagert.

30. Verfahren zur Herstellung des Hydrats in der Kristallform (c) des 4,4'-Bis-(2-sulfostyryl)-biphenyl-dinatrium Salzes gemäss Anspruch 4 und Tabelle 5, dadurch gekennzeichnet, dass man eine Suspension der Hydrate (i),(j) oder (p) mit mindestens ca. 5 Gew.% NaCl bezogen auf das Gesamtgewicht der Suspension und bei Raumtemperatur oder bis 100°C rührt und danach abkühlt.

31. Fliessfähige wässrige Formulierung enthaltend 30-50 Gew.% Aktivsubstanz des 4,4'-Bis-(2-sulfostyryl)-biphenyl-dinatrium Salzes, dadurch gekennzeichnet, dass das 4,4'-Bis-(2-sulfostyryl)-biphenyl-dinatrium Salz in der plättchenförmigen Kristallform 4,4'-Bis-(2-sulfostyryl)-biphenyl-dinatrium Salz in der plättchenförmigen Kristallform gemäss Anspruch 2 vorliegt.

32. Fliessfähige wässrige Formulierung gemäss Anspruch 31, dadurch gekennzeichnet, dass bei der Hauptmenge der plättchenförmigen Kristalle die Korngrösse über 10 µm liegt.

33. Viskose wässrige Formulierung enthaltend 30-50 Gew.% Aktivsubstanz des 4,4'-Bis-(2-sulfostyryl)-biphenyl-dinatrium Salzes, dadurch gekennzeichnet, dass das 4,4'-Bis-(2-sulfostyryl)-biphenyl-dinatrium Salz in der stäbchenförmigen Kristallform gemäss Anspruch 3 vorliegt.

34. Formulierung gemäss einem der Ansprüche 31 bis 33, dadurch gekennzeichnet, dass sie zusätzliche Formulierungshilfsmittel wie Dispergatoren, Gerüstbildner, Schutzkolloide, Stabilisatoren, Konservierungsmittel, Parfümierungsmittel sowie Sequestriermittel enthält.

35. Formulierung gemäss Anspruch 34, dadurch gekennzeichnet, dass sie 0,2 bis 5 Gew.% Propylenglykol enthält.

36. Formulierung gemäss Anspruch 34 oder 35, dadurch gekennzeichnet, dass sie 0,01 bis 2 Gew.% eines Heteropolysaccharides vom Xanthan-Typ enthält.

37. Formulierung gemäss einem der Ansprüche 34 bis 36, dadurch gekennzeichnet, dass sie 0,1 bis 1 Gew.% 1,2-Benzisothiazolin-3-on enthält.

38. Formulierung gemäss einem der Ansprüche 34 bis 37, dadurch gekennzeichnet, dass sie zusätzlich
a) 0,3-1 Gew.% Propylenglykol,
b) 0,1-0,5 Gew.% eines Heteropolysaccharides vom Xanthan-Typ,
c) 0,1-0,5 Gew.% 1,2-Benzisothiazolin-3-on und
d) Wasser enthält.

39. Verfahren zur Herstellung einer fliessfähigen wässrigen Formulierung des 4,4'-Bis-(2-sulfostyryl)-biphenyl-dinatrium Salzes, dadurch gekennzeichnet, dass man eine wässrige Suspension von 4,4'-Bis-(2-sulfostyryl)-biphenyl-dinatrium Salz mit einem Gehalt an Aktivsubstanz von höchstens 20 Gew.%, in der Form eines Hydrates mit dem Röntgenbeugungsdiagramm im wesentlichen wie in Tabelle 2,3,4 oder 5 für mehr als 4 Stunden bei 5 bis 45°C rührt und anschliessend aufkonzentriert.

40. Verwendung einer Formulierung entsprechend einem der Ansprüche 31 bis 39 zum optischen Aufhellen von Papier oder Textilmaterialien.

## Claims

1. A hydrate of the disodium salt or dipotassium salt of 4,4'-bis(2-sulfostyryl)biphenyl whose crystal form is characterised by an X-ray diffraction diagram which is essentially as in Table 1, 2, 3, 5 or 6 or a mixture of hydrates of the disodium salt or dipotassium salt of 4,4'-bis(2-sulfostyryl)biphenyl whose crystal forms are characterised by an X-ray diffraction diagram formed by additive superposition of the lines according to Tables 2 and 3, as shown in Table 4.

2. A hydrate in the platelet-like crystal form of the disodium salt of 4,4'-bis(2-sulfostyryl)biphenyl according to claim 1, characterised by an X-ray diffraction diagram which is essentially as in Table 1.

3. A hydrate in the rod-like crystal form of the disodium salt of 4,4'-bis(2-sulfostyryl)biphenyl according to claim 1, characterised by an X-ray diffraction diagram which is essentially as in Table 2 or 3 or a mixture of the two rod-like crystal forms corresponding to additive superposition of the lines according to Tables 2 and 3, as in Table 4.

4. A hydrate in the platelet-like crystal form c of the disodium salt of 4,4'-bis(2-sulfostyryl)biphenyl according to claim 1, characterised by an X-ray diffraction diagram which is essentially as in Table 5.

5. A hydrate of the dipotassium salt of 4,4'-bis(2-sulfostyryl)biphenyl according to claim 1, characterised by an X-ray diffraction diagram which is essentially as in Table 6.

6. A hydrate of the platelet-like crystal form (p) of the disodium salt of 4,4'-bis(2-sulfostyryl)biphenyl according to claim 2, of the formula (II) 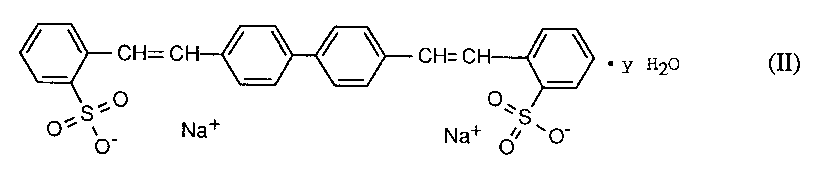 in which y is 10, 11 or 12.

7. A hydrate of the rod-like crystal form of the disodium salt of 4,4'-bis(2-sulfostyryl)biphenyl according to claim 3, of the formula (III) 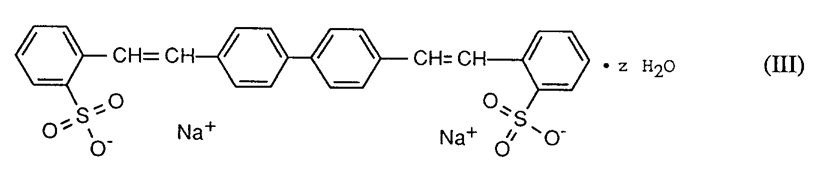 in which z is a number between 7 and 12.

8. A hydrate of the crystal form of the disodium salt of 4,4'-bis(2-sulfostyryl)biphenyl according to claim 4, of the formula (IV)

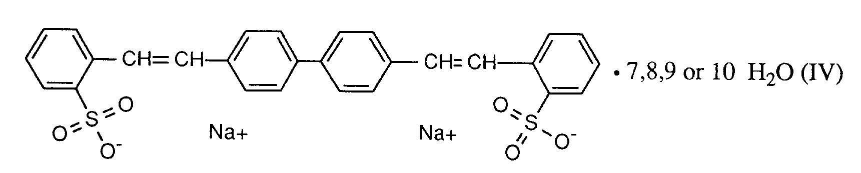

9. A hydrate of the dipotassium salt of 4,4'-bis(2-sulfostyryl)biphenyl according to claim 5, of the formula (V)

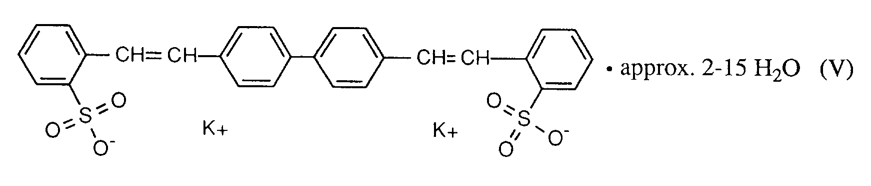

10. A process for the preparation of the platelet-like (p) crystal form according to claim 2, which comprises stirring an aqueous suspension of the disodium salt of 4,4'-bis(2-sulfostyryl)biphenyl having a maximum active substance content of 20 % by weight in the form of hydrates having an X-ray diffraction diagram which is essentially as in Table 2, 3 or 4, at 5 to 45°C and with a low shearing force for more than 4 hours.

11. A process for the preparation of the platelet-like (p) crystal form according to claim 2, which comprises adding platelet-like seed crystals to an aqueous suspension of any desired hydrate of the disodium salt of 4,4'-bis(2-sulfostyryl)biphenyl.

12. A process for the preparation of the platelet-like (p) crystal form according to claim 11, wherein an aqueous suspension having an active substance content of 30-50 % by weight is used.

13. A process for the preparation of the platelet-like (p) crystal form according to claim 11 or 12, wherein
a) the disodium salt of 4,4'-bis(2-sulfostyryl)biphenyl as prepared is homogenised,
b) seed crystals are added and
c) the mixture is stirred.

14. A process for the preparation of the platelet-like (p) crystal form according to any one of claims 11 to 13, wherein the seed crystal content is between 0.1 and 60 % by weight, relative to the overall content of the disodium salt of 4,4'-bis(2-sulfostyryl)biphenyl.

15. A process for the preparation of the compound of the platelet-like (p) crystal form according to claim 14, wherein the seed crystal content is between 1 and 50 % by weight, relative to the overall content of the disodium salt of 4,4'-bis(2-sulfostyryl)biphenyl.

16. A process for the preparation of the compound of the platelet-like (p) crystal form according to claim 15, wherein the seed crystal content is between 1 and 30 % by weight, relative to the overall content of the disodium salt of 4,4'-bis(2-sulfostyryl)biphenyl.

17. A process for the preparation of the platelet-like (p) crystal form according to any one of claims 11 to 16, wherein the process is carried out at a temperature of 5-45°C.

18. A process for the preparation of the platelet-like (p) crystal form according to claim 17, wherein the process is carried out at a temperature of 15-40°C.

19. A process for the preparation of the platelet-like (p) crystal form according to claim 13, wherein stirring is carried out intermittently.

20. A process for the preparation of the platelet-like (p) crystal form according to claim 11, wherein
a) the disodium salt of 4,4'-bis(2-sulfostyryl)biphenyl as prepared is suspended to give a suspension having an active substance content of 30-50 % by weight, and the suspension is homogenised,
b) 1-50 % by weight of platelet-like seed crystals, relative to the overall content of the disodium salt of 4,4'-bis(2-sulfostyryl)biphenyl, are added, and
c) the suspension is stirred at 15-40°C for 2-60 hours,
d) homogenised again and
e) deaerated by applying a vacuum.

21. A process for the preparation of the platelet-like (p) crystal form according to claim 11 or 12, wherein an aqueous suspension of the rod-like form according to claim 3 is seeded with 0.1 to 5 % by weight of the platelet-like form according to claim 2, relative to the overall active substance content.

22. A process for the preparation of the platelet-like (p) crystal form according to claim 21, wherein the seed crystals have an average size not exceeding 10 µm.

23. A process for the preparation of the platelet-like (p) crystal form according to claim 21 or 22, wherein conversion is effected without stirring.

24. A process for the preparation of the platelet-like (p) crystal form according to claim 2, which comprises mixing dried starting material with seed crystals and leaving the mixture at a relative humidity of 90-100 % and at 20-55°C for several days.

25. A process for the preparation of the rod-like crystal form according to claim 3 and Table 2, which comprises heating an aqueous suspension of any desired hydrate of the disodium salt of 4,4'-bis(2-sulfostyryl)biphenyl at a temperature of more than 70-130°C for 2-12 hours and then cooling it to room temperature.

26. A process for the preparation of the rod-like crystal form according to claim 3 and Table 3, which comprises heating the aqueous suspension containing 40 % by weight of any desired hydrate of the disodium salt of 4,4'-bis(2-sulfostyryl)biphenyl at a temperature of 95°C, in a closed vessel, and then cooling it to room temperature.

27. A process for the preparation of the rod-like crystal form according to claim 3 and Table 3, which comprises heating the aqueous suspension of any desired hydrate of the disodium salt of 4,4'-bis(2-sulfostyryl)biphenyl at a temperature of 42-70°C for 2 hours to 4 days and then cooling it to room temperature.

28. A process for the preparation of the rod-like crystal form according to claim 3 and Table 3, which comprises treating an aqueous suspension containing 40 % by weight of the disodium salt of 4,4'-bis(2-sulfostyryl)biphenyl in the platelet form for 4 hours at a temperature of 95°C, in a closed vessel, and then cooling it to room temperature.

29. A process for the preparation of the hydrate in the crystal form (c) of the disodium salt of 4,4'-bis(2-sulfostyryl)biphenyl according to claim 4 and Table 5, which comprises removing a portion of the water of an aqueous suspension of the hydrates in the (i) or (j) rod-like form or in the p platelet-form and then storing the suspension, depending on the established relative humidity of 52 % to around 100 %, for a period ranging from hours to days.

30. A process for the preparation of the hydrate in the crystal form (c) of the disodium salt of 4,4'-bis(2-sulfostyryl)biphenyl according to claim 4 and Table 5, which comprises stirring at least 5 % by weight of NaCl, based on the overall weight of the suspension, with a suspension of the hydrates (i),(j) or (p) at room temperature or up to 100°C, and then cooling.

31. A flowable aqueous formulation containing 30-50 % by weight of active substance, the disodium salt of 4,4'-bis(2-sulfostyryl)biphenyl, wherein the disodium salt of 4,4'-bis(2-sulfostyryl)biphenyl is present in the platelet-like crystal form according to claim 2.

32. A flowable aqueous formulation according to claim 31, wherein the particle size of the majority of the platelet-like crystals is above 10 µm

33. A viscous aqueous formulation containing 30-50 % by weight of active substance, the disodium salt of 4,4'-bis(2-sulfostyryl)biphenyl, wherein the disodium salt of 4,4'-bis(2-sulfostyryl)biphenyl is present in the rod-like crystal form according to claim 3.

34. A formulation according to any one of claims 31 to 33, which contains additional formulation auxiliaries, such as dispersants, builders, protective colloids, stabilisers, preservatives, perfuming agents and sequestering agents.

35. A formulation according to claim 34, which contains 0.2 to 5 % by weight of propylene glycol.

36. A formulation according to claim 34 or 35, which contains 0.01 to 2 % by weight of a heteropolysaccharide of the xanthan type.

37. A formulation according to any one of claims 34 to 36, which contains 0.1 to 1 % by weight of 1,2-benisothiazolin-3-one.

38. A formulation according to any one of claim 34 to 37, which additionally contains
a) 0.3-1 % by weight of propylene glycol,
b) 0.1-0.5 % by weight of a heteropolysaccharide of the xanthan type,
c) 0.1-0.5 % by weight of 1,2-benzisothiazolin-3-one and
d) water.

39. A process for the preparation of a flowable aqueous formulation of the disodium salt of 4,4'-bis(2-sulfostyryl)biphenyl, which comprises stirring an aqueous suspension of the disodium salt of 4,4'-bis(2-sulfostyryl)biphenyl having a maximum active substance content of 20 % by weight in the form of a hydrate having an X-ray diffraction diagram which is essentially as in Table 2,3,4 or 5 at 5 to 45°C for more than 4 hours and then concentrating it.

40. The use of a formulation according to any one of claims 31 to 39 for the fluorescent whitening of paper or textile materials.

## Revendications

1. Hydrate du sel disodique ou dipotassique de 4,4'-bis(2-sulfostyryl)-biphényle, dont la forme cristalline est caractérisée par un diagramme de diffraction de rayons X, essentiellement comme au tableau 1, 2, 3, 5 ou 6 ou bien un mélange d'hydrates du sel disodique de 4,4'-bis(2-sulfostyryl)-biphényle, dont les formes cristallines sont caractérisées par un diagramme de diffraction de rayons X, qui se forment essentiellement par la superposition additive des lignes selon les tableaux 2 et 3, comme indiqué au tableau 4.

2. Hydrate de cristaux, en forme de lamelles, du sel disodique de 4,4'-bis(2-sulfostyryl)-biphényle, selon la revendication 1, caractérisé par un diagramme de diffraction de rayons X, essentiellement comme au tableau 1.

3. Hydrate de cristaux, en forme de bâtonnets, du sel disodique de 4,4'-bis(2-sulfostyryl)-biphényle, selon la revendication 1, caractérisé par un diagramme de diffraction de rayons X, essentiellement comme au tableau 2 ou 3 ou bien un mélange des deux formes cristallines, en forme de bâtonnets, correspondant à une superposition additive des lignes selon les tableaux 2 et 3, comme indiqué au tableau 4.

4. Hydrate de cristaux de forme (c) du sel disodique de 4,4'-bis(2-sulfostyryl)-biphényle, selon la revendication 1, caractérisé par un diagramme de diffraction de rayons X, essentiellement comme au tableau 5.

5. Hydrate du sel disodique de 4,4'-bis(2-sulfostyryl)-biphényle, selon la revendication 1, caractérisé par un diagramme de diffraction de rayons X, essentiellement comme au tableau 6.

6. Hydrate de cristaux, en forme de lamelles (p), du sel disodique de 4,4'-bis(2-sulfostyryl)-biphényle, selon la revendication 2, de formule (II) 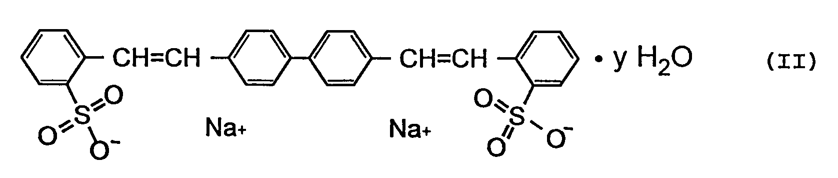 dans laquelle y représente un nombre compris entre 9 et 13.

7. Hydrate de cristaux, en forme de bâtonnets, du sel disodique de 4,4'-bis(2-sulfostyryl)-biphényle, selon la revendication 3, de formule (III) 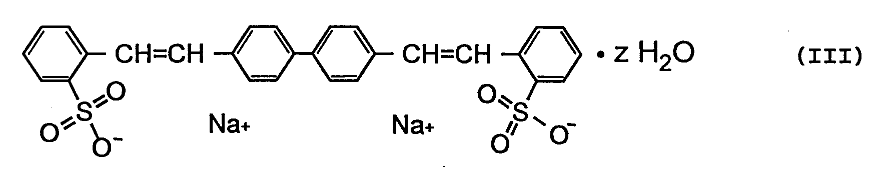 dans laquelle z représente un nombre compris entre 7 et 12.

8. Hydrate de cristaux du sel disodique de 4,4'-bis(2-sulfostyryl)-biphényle selon la revendication 4, de formule (IV)

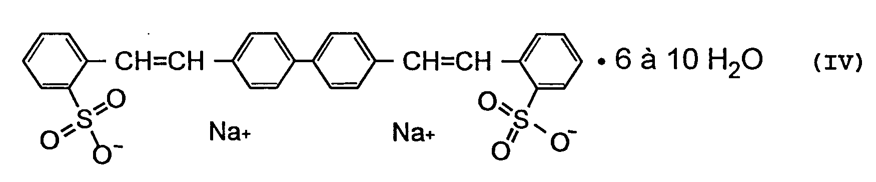

9. Hydrate du sel dipotassique de 4,4'-bis(2-sulfostyryl)-biphényle selon la revendication 5, de formule (V)

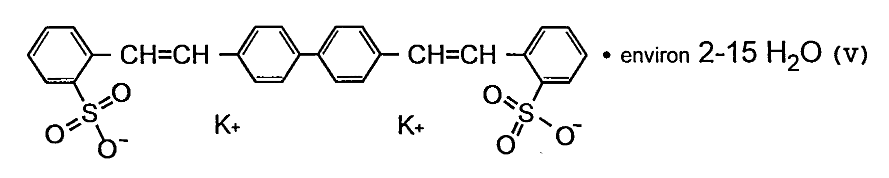

10. Procédé de préparation de cristaux en forme de lamelles (p), selon la revendication 2, caractérisé en ce qu'on agite, avec une faible force de cisaillement, pendant plus de 4 heures, à une température de 5 à 45°C, une suspension aqueuse du sel disodique de 4,4'-bis(2-sulfostyryl)-biphényle dont la teneur en substance active est au plus de 20% en poids et qui se présente sous forme d'hydrate et dont le diagramme de diffraction de rayons X est essentiellement comme au tableau 2, 3 ou 4.

11. Procédé de préparation de la forme cristalline (p) en forme de lamelles, selon la revendication 2, caractérisé en ce qu'on ajoute des cristaux d'ensemencement en forme de lamelles à une suspension aqueuse d'un hydrate quelconque du sel disodique de 4,4'-bis(2-sulfostyryl)-biphényle.

12. Procédé de préparation de la forme cristalline (p) en forme de lamelles, selon la revendication 11, caractérisé en ce qu'on utilise une suspension aqueuse dont la teneur en substance active est de 30 à 50% en poids.

13. Procédé de préparation de la forme cristalline (p) en forme de lamelles, selon la revendication 11 ou 12, caractérisé en ce que
a) on homogénéise le sel disodique de 4,4'-bis(2-sulfostyryl)-biphényle résultant de la préparation,
b) on y ajoute des cristaux d'ensemencement, et
c) on agite.

14. Procédé de préparation de la forme cristalline (p) en forme de lamelles, selon l'une des revendications 11 à 13, caractérisé en ce que la quantité de cristaux d'ensemencement est comprise entre 0,1 et 60% en poids calculé sur le poids total du sel disodique de 4,4'-bis(2-sulfostyryl)-biphényle.

15. Procédé de préparation du composé de forme cristalline (p) en forme de lamelles, selon la revendication 14, caractérisé en ce que la quantité de cristaux d'ensemencement est comprise entre 1 et 50% en poids calculé sur le poids total du sel disodique de 4,4'-bis(2-sulfostyryl)-biphényle.

16. Procédé de préparation du composé de forme cristalline (p) en forme de lamelles, selon la revendication 15, caractérisé en ce que la quantité de cristaux d'ensemencement est comprise entre 1 et 30% en poids calculé sur le poids total du sel disodique de 4,4'-bis(2-sulfostyryl)-biphényle.

17. Procédé de préparation de la forme cristalline (p) en forme de lamelles, selon l'une des revendications 11 à 16, caractérisé en ce qu'on travaille à une température allant de 5 à 45°C.

18. Procédé de préparation de la forme cristalline (p) en forme de lamelles, selon la revendication 17, caractérisé en ce qu'on travaille à une température allant de 15 à 40°C.

19. Procédé de préparation de la forme cristalline (p) en forme de lamelles, selon la revendication 13, caractérisé en ce qu'on agite de façon discontinue.

20. Procédé de préparation de la forme cristalline (p) en forme de lamelles, selon la revendication 11, caractérisé en ce que
a) on homogénéise le sel disodique de 4,4'-bis(2-sulfostyryl)-biphényle résultant de la préparation pour obtenir une suspension contenant de 30 à 50% en poids de substance active,
b) on y ajoute de 1 à 50% en poids de cristaux d'ensemencement en forme de lamelles, poids calculé sur le poids total du sel disodique de 4,4'-bis(2-sulfostyryl)-biphényle,
c) on agite à une température de 15 à 40°C pendant une durée de 2 à 60 heures,
d) on homogénéise encore une fois, et
e) on chasse l'air en utilisant une pression inférieure à la pression atmosphérique.

21. Procédé de préparation de la forme cristalline (p) en forme de lamelles, selon la revendication 11 ou 12, caractérisé en ce qu'on ensemence une suspension aqueuse en forme de bâtonnets, selon la revendication 3 avec une quantité allant de 0,1 à 5% en poids de forme en lamelles selon la revendication 2, poids calculé sur le Poids total de la substance active.

22. Procédé de préparation de la forme cristalline (p) en forme de lamelles, selon la revendication 21, caractérisé en ce que la dimension moyenne des cristaux d'ensemencement ne dépasse pas 10 µm.

23. Procédé de préparation de la forme cristalline (p) en forme de lamelles, selon la revendication 21 ou 22, caractérisé en ce que, lors de la transformation, on renonce à l'agitation.

24. Procédé de préparation de la forme cristalline (p) en forme de lamelles, selon la revendication 2, caractérisé en ce qu'on mélange la matière de départ séchée avec des cristaux d'ensemencement et qu'on les laisse en couche mince, pendant plusieurs jours, à une température de 20 à 55°C et à une humidité relative de l'air comprise entre 90 et 100%.

25. Procédé de préparation de la forme cristalline en forme de bâtonnets, selon la revendication 3 et tableau 2, caractérisé en ce qu'on chauffe à une température de 70-130°C pendant une durée de 2 à 12 heures une suspension aqueuse d'un hydrate quelconque du sel disodique de 4,4'-bis(2-sulfostyryl)-biphényle et qu'ensuite on refroidit à la température ambiante.

26. Procédé de préparation de la forme cristalline en forme de bâtonnets, selon la revendication 25, caractérisé en ce qu'on traite à une température de 95°C, dans un récipient fermé, pendant 4 heures, une suspension aqueuse contenant 40% en poids d'un hydrate quelconque du sel disodique de 4,4'-bis(2-sulfostyryl)-biphényle et qu'ensuite on refroidit à la température ambiante.

27. Procédé de préparation de la forme cristalline en forme de bâtonnets, selon la revendication 3 et tableau 3, caractérisé en ce qu'on chauffe à une température de 42 à 70°C pendant une durée de 2 heures à 4 jours la solution aqueuse d'un hydrate quelconque du sel disodique de 4,4'-bis(2-sulfostyryl)-biphényle et qu'ensuite on refroidit à la température ambiante.

28. Procédé de préparation de la forme cristalline en forme de bâtonnets selon la revendication 3 et tableau 3, caractérisé en ce qu'on traite, dans un récipient fermé, à une température de 95°C, pendant 4 heures, la solution aqueuse contenant 40% en poids du sel disodique de 4,4'-bis(2-sulfostyryl)-biphényle, sous forme de lamelles, et qu'ensuite on refroidit à la température ambiante.

29. Procédé de préparation de l'hydrate en forme de cristaux de forme (c) du sel disodique de 4,4'-bis(2-sulfostyryl)-biphényle, selon la revendication 4 et tableau 5, caractérisé en ce qu'on élimine une partie de l'eau d'une suspension aqueuse de l'hydrate en forme de bâtonnet (i) ou (j) ou sous forme de lamelles (p) et qu'ensuite on stocke pendant un temps correspondant à des heures ou à des jours, sous une humidité relative réglée entre 52% et 100%.

30. Procédé de préparation de l'hydrate en forme de cristaux de forme (c) du sel disodique de 4,4'-bis(2-sulfostyryl)-biphényle, selon la revendication 4 et tableau 5, caractérisé en ce qu'on agite une suspension d'hydrate (i), (j) ou (p) avec au moins 5% en poids de NaCl calculé sur le poids total de la suspension, l'agitation se faisant à la température ambiante ou à une température allant jusqu'à 100°C et qu'ensuite on refroidit.

31. Formulation aqueuse fluide renfermant de 30 à 50% en poids de substance active du sel disodique de 4,4'-bis(2-sulfostyryl)-biphényle, caractérisée en ce que le sel disodique de 4,4'-bis(2-sulfostyryl)-biphényle se présente sous forme cristalline en forme de lamelles selon la revendication 2.

32. Formulation aqueuse fluide selon la revendication 31, caractérisée en ce que la dimensions des particules de la majeure partie des cristaux en forme de lamelles dépasse 10 µm.

33. Formulation aqueuse visqueuse renfermant de 30 à 50% en poids de substance active du sel disodique de 4,4'-bis(2-sulfostyryl)-biphényle, caractérisée en ce que le sel disodique de 4,4'-bis(2-sulfostyryl)-biphényle se présente sous la forme cristalline en forme de bâtonnets, selon la revendication 3.

34. Formulation selon l'une des revendications 31 à 33, caractérisée en ce qu'elle contient en outre des adjuvants de formulation tels que des produits dispersants, des produits de soutien, des colloïdes protecteurs, des stabilisants, des agents de conservation, des parfums et des séquestrants.

35. Formulation selon l'une des revendications 34, caractérisée en ce qu'elle contient de 0,2 à 5% en poids de propylèneglycol.

36. Formulation selon l'une des revendications 34 ou 35, caractérisée en ce qu'elle contient de 0,01 à 2% en poids d'un hétéropolysaccharide du type Xanthan.

37. Formulation selon l'une des revendications 34, 35 ou 36, caractérisée en ce qu'elle renferme de 0,1 à 1% en poids de 1,2-benzisothiazoline-3-one.

38. Formulation selon l'une des revendications 34 à 37, caractérisée en ce qu'elle renferme en outre
a) de 0,3 à 1% en poids de propylèneglycol,
b) de 0,1 à 0,5% en poids d'un hétéropolysaccharide du type Xanthan,
c) de 0,1 à 0,5% en poids de 1,2-benzisothiazoline-3-one, et
d) de l'eau.

39. Procédé de préparation d'un formulation aqueuse fluide du sel disodique de 4,4'-bis(2-sulfostyryl)-biphényle, caractérisé en ce qu'on agite à une température allant de 5 à 45°C, pendant plus de 4 heures, une suspension aqueuse du sel disodique de 4,4'-bis(2-sulfostyryl)-biphényle ayant une teneur en substance active au plus de 20% en poids, sous la forme d'un hydrate dont le diagramme de diffraction de rayons X correspond pratiquement comme indiqué au tableau 2, 3, 4 ou 5.

40. Utilisation d'une formulation selon l'une des revendications 31 à 39 comme azurant optique pour le papier ou pour des matières textiles.
